# EUROPEAN PATENT APPLICATION

(11) **EP 2 510 881 A1**
(43) Date of publication of application: **17.10.2012**
(21) Application number: 11161858.3
(22) Date of filing: 11.04.2011
(51) Int. Cl.: A61B 10/00, C12M 1/26, C12M 1/30, C12M 1/02, B01F 13/08

(54) **Device and method for recovery of a collected specimen**

(71) Applicant: Qiagen GmbH, 40724 Hilden (DE)
(72) Inventor: Jeziorski, Markus, 40764, Langenfeld (DE)
(74) Representative: Gille Hrabal

(57) **Abstract**

The present invention relates to preparation of specimen or samples for analysis or diagnosis, and more particularly, to a device and method for recovery of a specimen/sample from a collecting material.
In order to improve the quality and sensitivity of subsequent analysis it is advantageous to enhance the recovery of collected specimen and thereby increase the concentration of the specimen to be analysed.
The objects and advantages of this invention are achieved by the apparatus and method of this invention, In accordance with this invention, the device includes a collection device (1) having a stem (2) and an absorbent head (3) for collecting a specimen, and a receptacle (7) for receiving the collection device with the collected specimen. The collection device contains at least one magnetic material (4). Further to that, a movable magnetic field for manipulating the collection device is provided.

## Description

The present invention relates to preparation of specimen or samples for analysis or diagnosis, and more particularly, to a device and method for recovery of a specimen/sample from a collecting material.

Methods and apparatuses are known for collecting and obtaining specimen, particularly for diagnostic and scientific examinations in biology and medicine. The range of collected specimen includes samples of bacteria and other micro-organism. Generally, such samples are taken by swabs with cotton or fibre pads, and the obtained specimen are transferred and released to a liquid for subsequent analysis or diagnosis. The transfer of the specimen collected in a swab to a liquid can be implemented by mechanical (vortex, ultrasound) or chemical means. Document US 4,789,639 discloses a liquid recovery device which transfers a sample to a liquid by wringing a cotton swab on a pad.

However, depending on the collected amount of specimen, the type of specimen, the utilized liquid solution and the chosen transfer method (automatic, manual), the transfer and recovery of the specimen, particularly of the substance of interest can be adversely affected. In order to improve the quality and sensitivity of subsequent analysis it is advantageous to enhance the recovery of collected specimen and thereby increase the concentration of the specimen to be analyzed. Furthermore, it is of interest to have a simple and standardized method and device for transferring and releasing collected specimen into a liquid for analysis, thus enhancing the overall reproducibility.

An object of this invention is to provide an improved device and method for collecting and recovering specimen for analysis or diagnosis.

The objects and advantages of this invention are achieved by the system, the collection device and the method according to the subject matter of the independent claims.

In accordance with claim 1, the system includes a collection device having a stem and an absorbent head for collecting a specimen, and a receptacle for receiving the collection device with the collected specimen. The collection device contains at least one magnetic material. Further to that, a movable magnetic field adapted to manipulate the magnetic material in the collection device is provided.

The present invention allows easier recovery of most of the collected specimen which otherwise would be retained by the collection device. The magnetic material inside the collection device can be manipulated by a movable magnetic field thereby releasing the collected specimen and/or handling the magnetic collection device. The collection device with the magnetic material can for example be moved and retained at the side of the receptacle to enable pipetting or further handling of the receptacle by fixing the collection device. The collection device can also be used inside hermetically closed receptacles, without the need for complicated seals. As no additional parts are needed for recovering the specimen, the risk of sample contamination is reduced thereby improving the integrity of the sample. Higher concentrations of analyte can be obtained leading to more sensitive analysis of the collected specimen.

In use, a collection device for example a swab with a cotton or polyester head is inserted into the cavity (oral, nasal, ocular or rectal, vaginal etc.) of the person. The collection device with the collected specimen is received in the receptacle which may comprise a releasable cap. The receptacle may be a usual sorbent tube containing an appropriate liquid like a universal transport medium (UTM), a culture medium or a nutrient medium. Preferably, the liquid comprises a lysis buffer which may be PCR compatible or chaotropic. The movable magnetic field manipulates the magnetic material in the collection device. This enables the separation of the specimen from the collection device, especially from the absorbent head in a standardized and repeatable manner. Furthermore, the release may be controlled for a defined amount of time increasing the repeatability of the recovery method. The preferred receptacle has a body portion, a closed bottom and an open top. Additionally, alternatively, the receptacle may comprise a cap or cover for removably closing the open top. The receptacle with the closed bottom and the open top improves the handling and storage of the collected specimen. The cap or cover further enables the sealed transport and storage thereby preventing contamination. After closing and sealing the receptacle with the cap the recovery of the collected specimen using the movable magnetic field can be carried out in a protected and sealed environment. The receptacle can be used for the transport of the specimen as well as the recovery of it. As the entire collected specimen is received in the receptacle the release of the collected specimen can be enhanced. Further, the hermetically sealed receptacle reduces contamination risks.

The magnetic material may be a ferromagnetic or ferrimagnetic material that responds to an applied magnetic field. Additionally, the collection device may contain more than one magnetic material to allow a more precise and flexible handling. The movable magnetic field is preferably located outside of the receptacle receiving the collection device and induces a magnetic field from outside of the receptacle thereby manipulating the magnetic material of the collection device accordingly. The manipulation of a magnetic material in the collection device by a movable magnetic field outside of the receptacle allows an easy and flexible arrangement of the movable magnetic field.

In an advantageous embodiment of this invention, at least one electromagnet is adapted to induce the movable magnetic field. The movable magnetic field may be implemented by an electromagnet which induces a magnetic field according to the electric current. The magnetic material of the collection device is manipulated by the induced magnetic field. The movable magnetic field may carry out any translational and/or rotational motion and thereby manipulate the magnetic material in the receptacle accordingly. The electromagnet preferably creates a rotating magnetic field which manipulates the magnetic material in the collection device causing it to spin. The magnetic material in the collection device can thereby be utilized as a magnetic stir bar enabling an improved recovery of the collected specimen. Additionally, alternatively, the electromagnet(s) may create a translatory magnetic field which manipulates the magnetic material in the collection device causing it to carry out a translatory motion, In this way, the collection device, particularly the absorbent head of the collection device, can be removed from the receptacle after recovery of the collected specimen, It is particularly advantageous to combine the rotatory and translatory motion in the movable magnetic field to manipulate the magnetic material in the collection device, In this way, it is possible to manipulate the location of the collection device in the receptacle as well as utilize the collection device as a magnetic stir bar. In addition, according to this invention, the movable magnetic field may also be implemented by a permanent magnet which may, for example, be rotated by a motor.

Advantageously, the above described recovery process is carried out with a constant rotation speed and/or a defined duration. By carrying out the recovery process with constant parameters, the release of the collected specimen is standardized leading to a consistent preparation of the samples.

In one embodiment of the invention, the collection device is adapted to be detachable. The collection device includes a detachment means which may be located along any part of the collection device, for example along the stem or shaft of the collection device. The detachment means may be a breakaway feature or a predetermined break zone for separating the absorbent head from the stem or the remaining part of the stem. Preferably, the detachment means is located near the absorbent head thereby only removing the absorbent head from the stem. The detached absorbent head is sized to fit into the receptacle, especially with regard to the cross section of the receptacle, and allow an unhindered motion inside of the receptacle. The detachable collection device allows for a compact absorbent head containing the collected specimen. The risk of sample contamination is also reduced, as the stem of the collection device which may be contaminated by the collection procedure, is not processed further with the collected specimen in the absorbent head. It is further possible to use a corresponding compact receptacle for receiving the absorbent head of the collection device with the collected specimen. By inserting the detached absorbent head in the receptacle it is ensured that the entire collected specimen is contained in the receptacle. The compact size of the absorbent head in conjunction with the corresponding receptacle further facilitates the storage and handling of the collected specimen.

In an advantageous embodiment of this invention, the magnetic material is located in the absorbent head of the collection device. The magnetic material may be a bar magnet or any other magnet formed to fit in the absorbent head of the collection device. Preferably, the length of the magnet is adapted to fit into the length of the absorbent head. The preferred stem of the collection device is formed to be hollow. This enables an easy-to-fabricate collection device. The magnetic material in the absorbent head of the collection device allows for an efficient recovery of the collected specimen in the absorbent head. It is thus possible to manipulate the part of the collection device which contains the entire collected specimen thereby optimizing the release of the collected specimen.

In another embodiment of the invention, the receptacle contains a liquid suspension. The liquid suspension may be adapted for the storage and/or transport of the collected specimen, for example a universal transport medium (UTM), Additionally, alternatively, the liquid suspension may be a culture or nutrient medium which can support the growth of microorganisms or cells. The use of a liquid suspension generally improves the release of the collected specimen. In addition, depending on the utilized suspension the transport, storage and/or growth of certain microorganisms/cells can be enhanced, particularly during the release of the collected specimen. By combining any of the effects of the utilized suspension with the recovery process, the overall process steps can be reduced and optimized.

In a preferred embodiment of the invention, the liquid suspension is a lysis buffer which may be PCR compatible or chaotropic. The lysis buffer may be chosen depending on the analysis for which the lysate will be used for. The lysis is preferably executed for 5 to 10 minutes during which a release of the collected specimen is also facilitated. By providing a lysis buffer in the receptacle receiving the collected specimen, it is possible to carry out the recovery of the collected specimen concurrently with the lysis of the specimen. The magnetic material of the collection device, preferably detached as a magnetic absorbent head, may be utilized as a magnetic stir bar thereby enabling a centrifuge process. The recovery step and the centrifuge step can thus be combined reducing the overall process steps and saving time.

The usage of a lysis buffer is particularly useful and advantageously employed in any system in which the sample has to be recovered and subsequently processed via lysis. A separate pipetting of the recovered specimen can be omitted and the lysis can be directly carried out for example in a sealed receptacle, thereby enabling fast sample processing and reducing the risk of sample contamination.

In another embodiment of this invention, said receptacle contains a liquid adapted for magnetic separation of the collected specimen. The magnetic separation may be implemented by magnetic beads that can bind to a desired target in the collected specimen like cells, microorganisms, nucleic acids, peptides, proteins and the like. The magnetic beads respond to a magnetic field, allowing the bound material to be separated from the rest of the sample. The separated material can then be utilized in a downstream analysis. The magnetic beads may respond to the magnetic material in the collection device. Additionally, alternatively, the magnetic beads may be manipulated by the movable magnetic field. The magnetic separation allows for a rapid and efficient sample preparation as magnetic separation and manipulation of the collection device with the magnetic material can be combined. Additional equipment for the preparation of the specimen or sample can thus be reduced.

It is particularly advantageous that the system, device and/or the method according to the invention is adapted for an at least partially automated system. The automated workflow enables a high throughput system. The storage, handling, recovery and processing of the specimen can thus be further improved.

In another advantageous embodiment of this invention, a control circuit for the movable magnetic field is provided. The control circuit may regulate the frequency, rotation and/or speed of the movable magnetic field. Additionally, alternatively, the amount of time may also be controlled by the control circuit. In use, a user may set a desired time for example for the rotation of the magnetic material in the collection device. The control circuit allows a precise control of the manipulation and/or agitation thereby enabling a recovery of the collected specimen in a standardized manner and increasing the reproducibility.

Other characteristics, advantages and potential applications of this invention are obvious from the description of further details and embodiments of this invention.

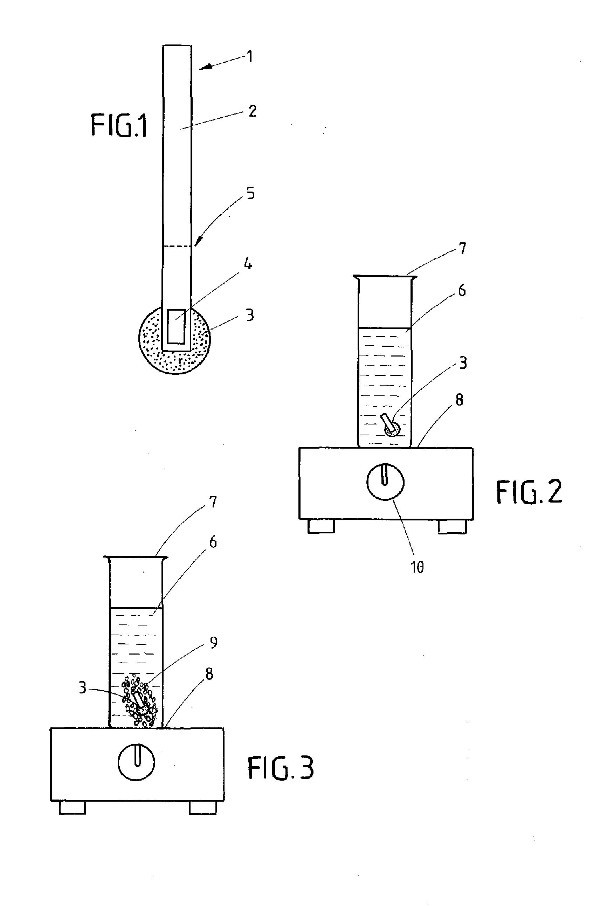

FIG. 1 is a schematic view of a collection device 1 having a stem 2 and an absorbent head 3 in accordance with one embodiment of the invention. The stem as illustrated is formed to be hollow on the inside. The absorbent head 3 may consist of a cotton, fibre or polyester pad in order to absorb a specimen. Inside the absorbent head a bar magnet 4 is arranged as the magnetic material. Alternatively, the stem 2 may be formed from a magnetic material or may comprise a magnetic material. The collection device includes a breakaway feature 5 to detach the absorbent head from the stem 2. By pressing the absorbent head 3 against a wall of a receptacle 7 the absorbent head 3 can be detached via the breakaway feature 5. At the same time, the absorbent head 3 can be submerged in a liquid suspension 6 in a receptacle 7 to recover the collected specimen from the absorbent head 3.

FIG.2 illustrates the system for preparing a specimen for analysis in accordance with one embodiment of the invention. The separated absorbent head 3 is immersed in a liquid suspension 7 which comprises a lysis buffer. The absorbent head 3 with the bar magnet 4 inside is manipulated by the movable magnetic field of the magnetic plate 8. The magnetic plate 8 arranged under the receptacle 7 induces a rotating magnetic field thereby causing the absorbent head 3 to stir and agitate the liquid suspension 6. The specimen collected in the absorbent head 3 is thus released into the liquid suspension 6 and concurrently a lysis is carried out. In order to control the recovery of the collected specimen in a standardized manner a control knob 10 for setting the amount of time is provided at the magnetic plate 8. Additionally, alternatively, the illustrated system can be implemented by a magnetic plate 8 that is aligned on the side of the receptacle 7. The magnetic plate 8 can thereby manipulate the absorbent head 3 to move vertically. In this way, the absorbent head 3 containing the magnetic material can be inserted and/or removed from the liquid suspension 6, for example after recovery of the specimen.

FIG. 3 illustrates the recovery process including magnetic separation in accordance with one embodiment of the invention. The liquid suspension contains magnetic beads 9 which specifically bind to a desired target like a protein or microorganism. As the magnetic beads 9 respond to a magnetic field, it is possible to separate the binded material from the rest of the sample either by the magnetic material inside the absorbent head 3 or the movable magnetic field located outside of the receptacle 7. The binded material can further be eluted in a suitable suspension for downstream processing.

The magnetic separation by magnetic beads 9 may be conducted after the recovery of the specimen. The magnetic beads 9 thereby bind to the desired target released in the liquid suspension 6. Alternatively, the magnetic separation may be conducted concurrently with the recovery of the collected specimen, In this case, the absorbent head containing the collected specimen is immersed in a suspension for magnetic separation. The absorbent head 3 with the magnetic material can be left in the liquid suspension 6 or removed from the liquid suspension 6 during the binding of the magnetic beads 9. The magnetic separation of the binded material can be implemented by the absorbent head 3 with the magnetic material as illustrated in FIG. 3 or by the movable magnetic field such as the magnetic plate 8.

Several studies were made to compare the release/recovery effect of a regular release/recovery method and a method in accordance with the invention, In one study, the collected specimen was released by the regular release method. A swab with an amount of a collected specimen in the absorbent head was held into a first receptacle containing a liquid suspension and manually agitated back and forth. Then, the same swab was immersed into a second receptacle with an identical liquid suspension and agitated in an identical manner. The agitation procedure was repeated in a third receptacle under equal conditions. Afterwards, the released specimen in each of the receptacles was analyzed. It was observed that the first receptacle contained a large amount of the collected specimen, but surprisingly even the subsequent receptacles contained a substantial amount of the specimen.

In another study, the recovery of an amount of specimen in an absorbent head was done in accordance with the invention. A swab with the collected specimen was immersed into a first receptacle containing a liquid suspension. The swab comprised a magnetic material and was manipulated by a rotating magnetic field provided under the receptacle. The rotating magnetic field was induced for 1 minute to agitate the swab in the liquid suspension with a consistent speed and for a defined duration. Then, the recovery step was equally repeated in a second and third receptacle under the same conditions. It was observed that the second and third receptacles contained only sparse amounts of the collected specimen.

These results demonstrate the disadvantages of a manual release/recovery process. The manual release process is subject to considerable fluctuations depending on various uncontrollable factors such as the agitation by a user. Different amounts of the recovered specimen lead to variations in the analysis and diagnosis.

Further, the studies demonstrate the advantages of the recovery process in accordance with the invention. The process is carried out in a standardized manner and thereby improves the reproducibility of the release/recovery process. Adverse influences occuring in a manual recovery process are thereby eliminated. The recovery process is also much more efficient in releasing the collected specimen from the absorbent head of the swab. By detaching the absorbent head from the rest of the collection device, it is furthermore guaranteed that the entire collected specimen is contained in the receptacle and the release efficiency is improved. In addition, the magnetic absorbent head may be utilized as a magnetic stir bar allowing it to be used as a centrifuge means for a lysis. Thus, the release/recovery process is carried out concurrent with a lysis process reducing the overall preparation steps.

## Claims

1. A system for preparing a specimen for analysis comprising a collection device (1) having a stem (2) and an absorbent head (3) for collecting a specimen, wherein the collection device (1) contains at least one magnetic material (4), a receptacle (7) for receiving the collection device with the specimen, a movable magnetic field (8) adapted to manipulate the magnetic material (4) in the collection device (1).

2. A system according to the preceding claim, **characterised in that** said collection device (1) is adapted to be detachable.

3. A system according to any of the preceding claims, **characterised in that** said magnetic material (4) is located in the absorbent head (3) of the collection device (1).

4. A system according to any of the preceding claims, **characterised in that** said movable magnetic field (8) contains at least one electromagnet.

5. A system according to any of the preceding claims, **characterised in that** said movable magnetic field (8) is adapted to carry out a rotatory and/or translatory motion.

6. A system according to any of the preceding claims, **characterised in that** said receptacle (7) contains a liquid suspension (6).

7. A system according to the preceding claim, **characterised in that** said liquid suspension (6) comprises a lysis buffer.

8. A system according to any of the preceding claims, **characterised in that** said receptacle (7) contains a liquid suspension adapted for magnetic separation of the collected specimen.

9. A collection device for preparing a specimen for analysis comprising a stem (2), an absorbent head (3) and at least one magnetic material (4) provided in the collection device (1).

10. A collection device according to the preceding claim, **characterised in that** said collection device (1) is detachable, preferably separating the absorbent head (3) from the stem (2).

11. A collection device according to any of claims 9-1 0, **characterised in that** said magnetic material (4) is located in the absorbent head (3).

12. A collection device according to any of claims 9-1 1 , **characterised in that** said stem (2) is formed to be hollow and/or formed from of a magnetic material.

13. A method for preparing a specimen for analysis comprising collecting a specimen by a collection device (1), the collection device comprising an absorbent head (3) and at least one magnetic material (4), releasing the collected specimen from the collection device (1) into a receptacle (7), whereby the collection device (1) is manipulated in the receptacle (7) by a movable magnetic field (8).

14. A method according to the preceding claim, **characterised in that** the release of the collected specimen is combined with a lysis, preferably by means of a lysis buffer.

15. A method according to one of the two preceding claims, **characterised in that** the specimen is separated by magnetic separation, particularly by magnetic beads (9).
